Europäisches Patentamt

European Patent Office

Office européen des brevets

(11) Veröffentlichungsnummer: **0 106 960**
**B1**

# (12) EUROPÄISCHE PATENTSCHRIFT

(45) Veröffentlichungstag der Patentschrift:
04.02.87

(21) Anmeldenummer: **83107932.2**

(22) Anmeldetag: **11.08.83**

(51) Int. Cl.⁴: **A 61 F 2/30**, A 61 M 3/00

(54) **Einweg-Knochenzementspritze.**

(30) Priorität: **21.10.82 CH 6118/82**

(43) Veröffentlichungstag der Anmeldung:
**02.05.84 Patentblatt 84/18**

(45) Bekanntmachung des Hinweises auf die Patenterteilung:
**04.02.87 Patentblatt 87/6**

(84) Benannte Vertragsstaaten:
**AT DE FR IT**

(56) Entgegenhaltungen:
**EP-A-0 006 430**
**US-A-4 331 188**

(73) Patentinhaber: **GEBRÜDER SULZER AKTIENGESELLSCHAFT, Zürcherstrasse 9, CH-8401 Winterthur (CH)**
Patentinhaber: **Protek AG, Stadtbachstrasse 64, CH-3001 Bern (CH)**

(72) Erfinder: **Müller, Maurice E., Prof.Dr.- med., Inselspital, CH- 3010 Bern (CH)**
Erfinder: **Frey, Otto, Walrütistrasse 56, CH- 8404 Winterthur (CH)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf (DE)**

EP 0 106 960 B1

## Beschreibung

Die Erfindung betrifft eine Einweg-Zementspritze zum Einfüllen von Knochenzement in operativ vorbereitete Knochenhohlräume, bestehend aus einem Kolben und einem Zylinderrohr, an dessen einem Ende ein Düsenkörper mit einem Düsenrohr relativ engen Querschnitts angeordnet ist, in dem ein Stössel kolbenartig verschiebbar ist.

Zementspritzen der vorstehend genannten Art sind bekannt (US-A-4,331,188); sie dienen dazu, das Mischen und Austragen des pastösen Knochenzements sowie das Füllen des engen Düsenrohrs auf einfache Weise zu ermöglichen. Nach dem Mischen des Zements in dem Zylinderrohr wird mit Hilfe eines Kolbens bei dieser Konstruktion daher zunächst das Düsenrohr gefüllt und anschliessend mit einem - an seinen Querschnitt angepassten - Stössel der Zement in den Körper eingespritzt.

In der EP-A- 0 006 430 ist eine ähnliche Zementspritze beschrieben und gezeigt, bei der an den mit einem Kolben beaufschlagten Zylinderrohr ein Düsenrohr engen Querschnitts ansetzbar ist; es ist jedoch kein im Düsenrohr verschiebbarer Stössel vorhanden. Mit dieser Konstruktion soll das Austragen des Knochenzements, der in dem Zylinderrohr gemischt wird, erleichtert werden.

Im Gegensatz zu den mit den beiden bekannten Konstruktionen angestrebten Zielen besteht die der vorliegenden Erfindung zugrundeliegende Aufgabe darin, eine Knochenzementspritze zu schaffen, mit der es möglich ist, einmal bei relativ geringem Druck eine relativ grosse Menge Knochenzement auszutragen, und zum anderen bei relativ hohem Druck - ohne Anwendung wesentlich erhöhter Kräfte - eine kleine Menge in den Knochen einzubringen. Die beiden Verwendungsarten sollen dabei sowohl jede für sich als auch in Kombination - d.h. zuerst eine und im gleichen Arbeitsprozess anschliessend die andere - angewendet werden können. Mit den vorstehend beschriebenen, bekannten Zementspritzen ist es nicht möglich, sowohl eine "Niederdruck"- als auch eine "Hochdruck"-Einspritzung vorzunehmen, bei der der Druck etwa um das Zwanzigfache erhöht ist.

Erfindungsgemäss wird die geschilderte Aufgabe gelöst durch einen das Zylinderrohr abschliessenden Zwischenboden mi einer Spritzdüse, deren lichte Weite den Düsenquerschnitt des Düsenrohres umfasst, ferner durch Mittel, mit deren Hilfe der Düsenkörper während des Gebrauchs an das Zylinderrohr ansetzbar bzw. von ihm abnehmbar ist.

Bei der neuen Konstruktion wird die relativ grosse Menge Zement - bei abgenommenem Düsenkörper - durch die Spritzdüse des Zwischenbodens hindurch ausgetragen; weiterhin erfüllt das Zylinderrohr mit der Spritzdüse in bekannter Weise auch die Aufgabe, den Düsenkörper bzw. das Düsenrohr mit Knochenzement zu füllen. Für einen "Hochdruck"-Eintrag von Knochenzement in den Knochenhohlraum wird das gefüllte Düsenrohr mit Hilfe des Stössel entleert. Dabei ist es möglich, den Stössel - bei an das Zylinderrohr angesetztem Düsenkörperdurch das Zylinderrohr und die Spritzdüse hindurch in das Düsenrohr zu führen; man kann jedoch auch den Düsenkörper vom Zylinderrohr, an dem er beispielsweise mit Hilfe eines Bajonett-Verschlusses befestigt ist, lösen und als separate Spritze gebrauchen.

Vorteilhafterweise kann zwischen dem Düsenrohr des Düsenkörpers und der Spritzdüse des Zwischenbodens ein Vorratsvolumen vorhanden sein. Soll die neue Zementspritze in einem kombinierten Arbeitsprozess sowohl als "Hochdruck"- als auch als "Niederdruck"-Spritze verwendet werden, so ist es zweckmässig, wenn das Zylinderrohr den Inhalt vonmehr als einer Standardpackung des Knochenzements, vorzugsweise zwei Standardpackungen, aufnimmt; eine solche Standardpackung enthält bekanntlich etwa 50 - 60 cm³, im allgemeinen etwa 55 cm³, Knochenzement.

Weiterhin kann der Stössel mit einer tellerartigen Auflageplatte an den im Zylinderrohr verschiebbaren Kolben ansetzbar und im Zylinderrohr versenkbar sein. Das bringt den Vorteil, dass die gesamte Knochenzementspritze ohne Lösen des Bajonettverschlusses entleert werden kann.

Da die neue Knochenzementspritze für einmaligen Gebrauch bestimmt ist, besteht sie aus einem für solche Spritzen üblichen Kunststoff, beispielsweise Polymethylpenten (TPX); ihre Teile werden dabei in erster Linie im Spritzguss-Verfahren oder als Pressteile hergestellt.

Im folgenden wird die Erfindung anhand eines Ausführungsbeispiels im Zusammenhang mit der Zeichnung näher erläutert.

Fig. 1 zeigt eine Ansicht des Zylinderrohres für die neue Zementspritze;

Fig. 2 gibt eine Ansicht von oben auf Fig. 1 wieder und verdeutlicht den Zwischenboden mit der Spritzdüse;

Fig. 3 und 4 zeigen den - teilweise im Schnitt wiedergegebenen - Düsenkörper mit dem Düsenrohr und den zugehörigen Stössel, während

Fig. 5 in einem Längsschnitt die neue Zementspritze im zusammengebauten Zustand wiedergibt.

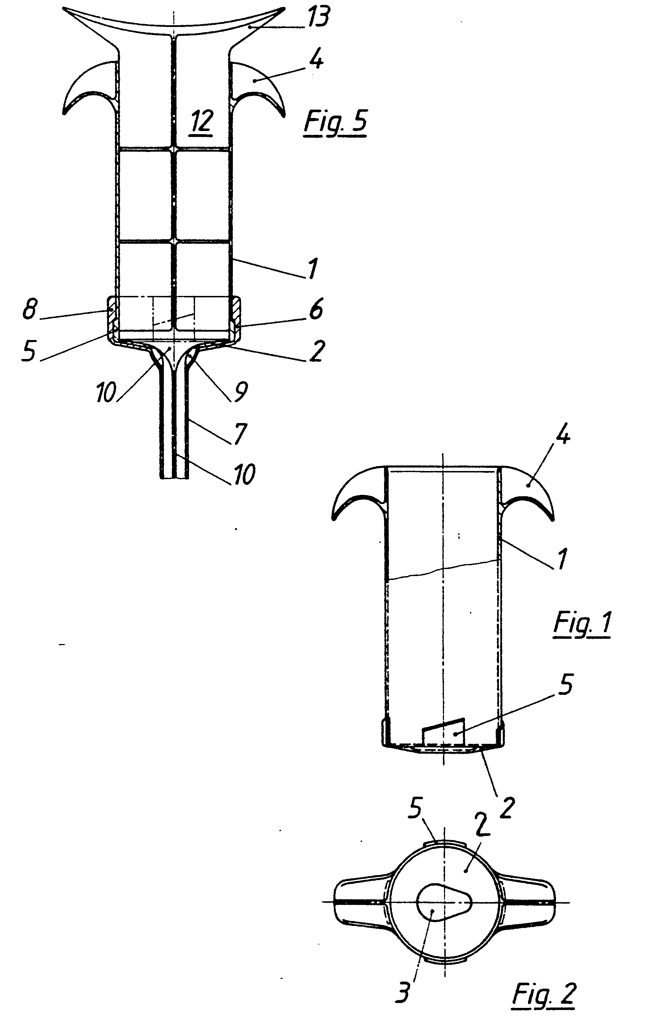

Das Zylinderrohr 1 (Fig. 1), dessen Volumen - mit beispielsweise 125 cm³ - so ausgelegt ist, dass es zwei Standard-Packungen Knochenzement aufnehmen kann, ist unten durch einen in der Mitte leicht konisch abgesenkten Zwischenboden 2 abgeschlossen; im wesentlichen im Zentrum des Zwischenbodens 2 ist eine Spritzdüse 3 vorhanden, die in grober Ännäherung der Quetschnittsform eines Schaftes für eine Femurkopfprothese entspricht.

Während am oberen Ende des Zylinderrohrs 1 als Widerlager für die Finger zwei seitliche Lappen 4 angeordnet sind, ist das untere Ende mit vier gleichmässig über den Umfang verteilten rechteckigen Verstärkungen 5 versehen, deren obere Kanten in der gleichen Richtung abgeschrägt sind.

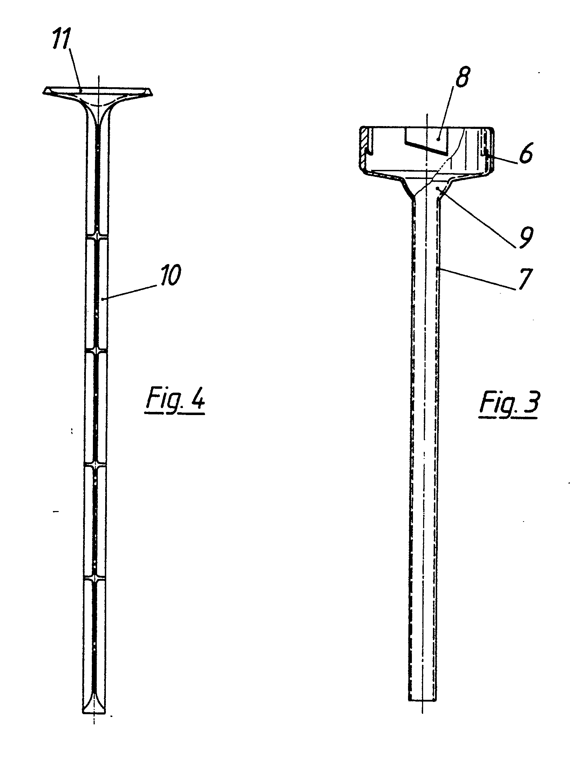

Die Verstärkungen 5 sind Teil eines Bajonettverschlusses, mit dem ein Düsenkörper 6 (Fig. 3) lösbar an das Zylinderrohr 1 angesetzt werden kann. In diesem Körper 6, der nach unten über ein kleines Vorratsvolumen 9 von wenigen cm³ in ein Düsenrohr 7 übergeht, weist daher an seinem oberen Ende entsprechend gegengleiche nach innen gerichtete Vorsprünge 8 auf.

Der lichten Weite des Düsenrohrs 7 angepasst ist ein Stössel 10, der als Kolben in das Düsenrohr 7 bei Verwendung der Spritze zur "Hochdruck"-Einspritzung eingeschoben wird. Der Stössel 10 endet oben in einer tellerartigen Auflageplatte 11 für einen Finger bzw. den Daumen des Benutzers.

Der Durchmesser der Platte 11 ist auf denjenigen eines im Zylinderrohr 1 verschiebbaren Kolbens 12 abgestimmt, so dass der Stössel 10 gegebenenfalls an den Kolben 12 angesetzt werden kann, um ihn beispielsweise mit Hilfe dieses Kolbens 12 zu führen.

Ähnlich wie das Zylinderrohr 1, weist der Kolben 12 Handgriffe 13 auf, die im Zusammenwirken mit dem Lappen 4 die Handhabung der Zementspritze erleichtern.

## Patentansprüche

1. Einweg-Zementspritze zum Einfüllen von Knochenzement in operativ vorbereitete Knochenhohlräume, bestehend aus einem Kolben (12) und einem Zylinderrohr (1), an dessen einem Ende ein Düsenkörper (6) mit einem Düsenrohr (7) relativ engen Querschnitts angeordnet ist, in dem ein Stössel (10) kolbenartig verschiebbar ist, gekennzeichnet durch einen das Zylinderrohr (1) abschliessenden Zwischenboden (2) mit einer Spritzdüse (3), deren lichte Weite den Düsenquerschnitt des Düsenrohrs (7) umfasst, ferner durch Mittel, mit deren Hilfe der Düsenkörper (6) während des Gebrauchs an das Zylinderrohr (1) ansetzbar bzw. von ihm abnehmbar ist.

2. Zementspritze nach Anspruch 1, dadurch gekennzeichnet, dass zwischen dem Düsenrohr (7) des Düsenkörpers (6) und der Spritzdüse (3) des Zwischenbodens (2) ein Vorratsvolumen (9) vorhanden ist.

3. Zementspritze nach Anspruch 1 oder 2, dadurch gekennzeichnet, dass das Zylinderrohr (1) den Inhalt von mehr als einer Standardpackung des Knochenzements, vorzugsweise etwa zwei Standardpackungen, aufnimmt.

4. Zementspritze nach einem der Ansprüche 1 bis 3, dadurch gekennzeichnet, dass der Stössel (10) mit einer tellerartigen Auflageplatte (11) an den im Zylinderrohr (1) verschiebbaren Kolben (12) ansetzbar und im Zylinderrohr (1) versenkbar ist.

## Claims

1. A one-trip cement syringe for injecting bone cement into operatively prepared bone cavities, the injector comprising a piston (12) and a cylindrical tube (1) having at one end a nozzle member (6) having a relatively narrow cross-section nozzle tube (7) in which a pusher (10) can move piston-fashion, characterised by: an intermediate member (2) which closes off the cylindrical tube (1) and which has an injection nozzle (3), the internal width thereof comprising the nozzle cross-section of the nozzle tube (7); and by means enabling the nozzle member (6) to be fitted to or removed from the cylindrical tube (1) in use.

2. A syringe according to claim 1, characterised in that a supply volume (9) is present between the nozzle tube (7) of the nozzle member (6) and the syringe nozzle (3) of the intermediate member (2).

3. A syringe according to claim 1 or 2, characterised in that the cylindrical tube (1) receives the contents of more than one standard pack of bone cement, preferably approximately two standard packs.

4. A syringe according to any of claims 1 - 3, characterised in that the pusher (10) has a dished support plate (11) by way of which the pusher (10) can be fitted to the piston (12) movable in the tube (1) and can be lowered therein.

## Revendications

1. Pompe à injection de ciment à voie unique destinée à remplir de ciment osseux des cavités osseuses préparées chirurgicalement, constituée par un piston (12) et un tube cylindrique (1) à l'une des extrémités duquel on dispose un corps de buse (6) ayant un tube de buse (7) d'une section transversale relativement étroite dans lequel peut se déplacer à la façon d'un piston un poussoir (10), caractérisée par un corps intermédiaire (2) fermant le tube cylindrique (1) et ayant une buse d'injection (3) dont la largeur interne couvre la section transversale de buse du tube à buse (7), ainsi que par des moyens à l'aide desquels le corps de buse (6) peut être rapporté pendant l'emploi sur le tube cylindrique (1) ou enlevé de celui-ci.

2. Pompe à injection de ciment selon la revendication 1, caractérisée en ce que, entre le tube à buse (7) du corps à buse (6) et la buse d'injection (3) du fond intermédiaire (2), se trouve un volume de réserve (9).

3. Pompe à injection de ciment selon la revendication 1 ou 2, caractérisée en ce que le tube cylindrique (1) reçoit le contenu de plus d'un paquet standard de ciment osseux, de préférence environ deux paquets standard.

4. Pompe à injection de ciment selon l'une des revendications 1 à 3, caractérisée en ce que le poussoir (10) peut être rapporté avec une plaque d'application (11) du type plateau sur le piston (12) pouvant se déplacer dans le tube cylindrique (1) et peut être descendu dans le tube cylindrique (1).

Fig. 5

Fig. 1

Fig. 2

11

8

6

10

9

7

_Fig. 4_

_Fig. 3_